# EUROPEAN PATENT SPECIFICATION

(11) **EP 0 652 787 B1**
(45) Date of publication and mention of the grant of the patent: **05.02.2003**
(21) Application number: 93918414.9
(22) Date of filing: 27.07.1993
(51) Int. Cl.: A61M 29/02

(54) **LOW PROFILE CATHETER WITH EXPANDABLE OUTER TUBULAR MEMBER**
GERINGER QUERSCHNITT-KATHETER MIT DEHNBAREM AUSSENROHR
CATHETER EXTRAPLAT AVEC ELEMENT TUBULAIRE EXTERNE EXPANSIBLE

(30) Priority: 28.07.1992 US 921304
(43) Date of publication of application: 17.05.1995
(73) Proprietor: ADVANCED CARDIOVASCULAR SYSTEMS, INC., Santa Clara, California 95052-8167 (US)
(72) Inventor: MUNI, Ketan, P., San Jose, CA 95136 (US); SIRHAN, Motasim, M., Sunnyvale, CA 94087 (US)
(74) Representative: Pendlebury, Anthony
(86) International application number: US9307043
(87) International publication number: WO94002197

(56) References cited:
- EP-A- 0 304 258
- EP-A- 0 540 858
- US-A- 4 406 656
- US-A- 4 748 982
- US-A- 4 892 519

## Description

### BACKGROUND OF THE INVENTION

This invention generally relates to intraluminal catheters, such as balloon dilatation catheters used in percutaneous transluminal coronary angioplasty (PTCA). The invention also relates to a method of forming an outer tubular member for an intraluminal catheter.

PTCA is a widely used procedure for the treatment of coronary heart disease. In this procedure, a balloon dilatation catheter is advanced into the patient's coronary artery and the balloon on the catheter is inflated within the stenotic region of the patient's artery to open up the arterial passageway and thereby increase the blood flow therethrough. To facilitate the advancement of the dilatation catheter into the patient's coronary artery, a guiding catheter having a preshaped distal tip is first percutaneously introduced into the cardiovascular system of a patient by the Seldinger technique through the brachial or femoral arteries. The catheter is advanced therein until the preshaped distal tip of the guiding catheter is disposed within the aorta adjacent the ostium of the desired coronary artery. The guiding catheter is twisted or torqued from the proximal end, which extends out of the patient, to guide the distal tip of the guiding catheter into the ostium. A balloon dilatation catheter may then be advanced through the guiding catheter into the patient's coronary artery until the balloon on the catheter is disposed within the stenotic region of the patient's artery. The balloon is inflated to open up the arterial passageway and increase the flow of blood.

One type of catheter frequently used in PTCA procedures is an over-the-wire type balloon dilatation catheter. Commercially available over-the-wire type dilatation catheters include the SIMPSON ULTRA LOW PROFILE™, the HARTZLER ACX®, the HARTZLER ACX II®, the PINKERTON .018™ and the ACS TEN™ balloon dilatation catheters sold by the assignee of the present invention, Advanced Cardiovascular Systems, Inc. (ACS). When using an over-the-wire dilatation catheter, a guidewire is usually inserted into an inner lumen of the dilatation catheter before it is introduced into the patient's vascular system and then both are introduced into and advanced through the guiding catheter to its distal tip which is seated within the ostium of the desired coronary artery. The guidewire is first advanced out the seated distal tip of the guiding catheter into the desired coronary artery until the distal end of the guidewire extends beyond the lesion to be dilatated. The dilatation catheter is then advanced out of the distal tip of the guiding catheter into the patient's coronary artery, over the previously advanced guidewire, until the balloon on the distal extremity of the dilatation catheter is properly positioned across the lesion to be dilatated. Once properly positioned across the stenosis, the balloon is inflated one or more times to a predetermined size with radiopaque liquid at relatively high pressures (*e.g*., generally 405-1215 kPa (4-12 atmospheres)) to dilate the stenosed region of a diseased artery. After the inflations, the balloon is finally deflated so that the dilatation catheter can be removed from the dilated stenosis and blood flow will resume.

Fixed-wire type dilatation catheter system are also utilized very frequently in PTCA procedures. This type of dilatation catheter has a guidewire or guiding member secured within the catheters and it provides a low profile, i.e. small transverse dimensions, because there is no inner tubular member which is characteristic of commercially available over-the-wire dilatation catheters. Commercially available fixed-wire dilatation catheters include the HARTZLER EXCEL®, the HARTZLER LPS® and the SLALOM™ dilatation catheters sold by ACS.

Another type of dilatation catheter, the rapid exchange type catheter, was introduced by ACS under the trademark ACS RX® Coronary Dilatation Catheter. It is described and claimed in U.S. Patent 5,040,548 (Yock), U.S. Patent 5,061,273 (Yock) and U.S. Patent 4,748,982 (Horzewski *et al*.). This dilatation catheter has a short guidewire receiving sleeve or inner lumen extending through a distal portion of the catheter. The sleeve or inner lumen extends proximally from a first guidewire port in the distal end of the catheter to a second guidewire port in the catheter spaced proximally from the inflatable member of the catheter. A slit may be provided in the wall of the catheter body which extends distally from the second guidewire port, preferably to a location proximal to the proximal end of the inflatable balloon. The structure of the catheter allows for the rapid exchange of the catheter without the need for an exchange wire or adding a guidewire extension to the proximal end of the guidewire. This catheter has been widely praised by the medical profession and it has met with much success in the market place because of the advantages of its unique design.

The perfusion type dilatation catheter was another type of dilatation catheter introduced into the market place by ACS. This catheter, which can take the form of an over-the-wire, fixed-wire or rapid exchange type catheter, has one or more perfusion ports proximal to the dilatation balloon in fluid communication with an guidewire receiving inner lumen extending to the distal end of the catheter. A plurality of perfusion ports are preferably provided in the catheter, distal to the balloon, which are also in fluid communication with the inner lumen extending to the distal end of the catheter. When the balloon of this dilatation catheter is inflated to dilate a stenosis, oxygenated blood in the artery or the aorta or both, depending upon the location of the dilatation catheter within the coronary anatomy, is forced to pass through the proximal perfusion ports, through the inner lumen of the catheter and out the distal perfusion ports. The catheter provides oxygenated blood downstream from the inflated balloon to minimize ischemic conditions in tissue distal to the balloon. The perfusion of blood distal to the inflated balloon allows for long term dilatations, e.g. 30 minutes or even several hours or more. This catheter has likewise been highly praised by the medical profession and has met with much commercial success. Commercially available perfusion type dilatation catheters include the STACK PERFUSION® and the ACS RX PERFUSION"' dilatation catheters which are sold by ACS.

A continual effort has been made in the field of intravascular catheters, particularly angioplasty catheters, to reduce the transverse dimensions or profile of such catheters without detrimentally affecting the pushability and other characteristics of the catheters, particularly in the distal portion of the catheters. A balloon dilatation catheter with a reduced profile and an increase or no loss in pushability allow an the catheter to be advanced much further into a patient's vasculature and to cross much tighter lesions.

Despite the many technical advances in these areas, the need for intravascular catheters having even lower distal profiles with excellent flexibility and pushability remains. The present invention satisfies these and other needs.

EP-A-0304258 includes a catheter having the features of the precharacterising portion of claim 1.

### SUMMARY OF THE INVENTION

The present invention is directed to a dilatation catheter having a low profile catheter shaft, particularly in the distal portion thereof, with improved flexibility and pushability.

According to a first aspect of the present invention there is provided an intraluminal catheter comprising an elongated catheter shaft having proximal and distal portions, the shaft having an inner tubular member and an outer tubular member which is disposed about said inner tubular member to define therebetween an annular inner lumen which extends through the proximal portion of the catheter shaft wherein the distal portion of the outer tubular member has a first configuration in which it forms an inflation lumen, and a second configuration in which it is collapsed about the inner tubular member and an inflatable member at a distal end of the distal portion of the catheter shaft, wherein said inflation lumen is in fluid communication with the interior of the inflatable member and the annular lumen characterized in that the distal portion of the outer tubular member expands substantially elastically from said second configuration upon inflation to said first configuration in a first pressure range, and expands very little in said first configuration in a second pressure range higher than the first pressure range.

According to a second aspect of the present invention there is provided a method of forming an outer tubular member for an intraluminal catheter in accordance with the invention, the outer tubular member having an expandable distal portion and an inflatable member at a distal end of the distal portion, the method comprising: a) extruding through a die an elongated tubular member formed of a polymer material selected from the group consisting of sodium, lithium and zinc ionomers and blends thereof at elevated temperatures; b) quenching the extruded tubular member as it exits from the die; c) irradiating first and second portions of the tubular member which are to be formed into the expandable distal portion and the inflatable member respectively, to cross-link the polymer material thereof; d) heat treating the first and second portions of the tubular member at a temperature about 75°C above or about 75°C below the crystalline melting point of the polymer material; and e) expanding by inflation the first and second portions and then cooling and heat shrinking the first and second portions, the second portion being expanded to a larger diameter than the first portion.

The catheter shaft of the invention generally includes, at least in a distal portion thereof, an expandable outer tubular member defining an inner lumen and an inflatable member distal to the expandable outer tubular member. The outer tubular member in the distal section is formed of polymer material which allows it to expand to a larger diameter in an elastic mode to facilitate the flow of inflation fluid to the interior of the inflatable member on the distal end of catheter shaft when it is inflated. Upon deflation, the expanded outer tubular member contracts by elastic recoil to a diameter substantially smaller than the expanded diameter thereof. The distal section of the outer tubular member expands significantly when the internal pressure is within a first pressure range. However, when the internal pressure is within a second higher pressure range the expansion is very limited because the distal section is relatively noncompliant. The expansion at the second higher pressure level, while limited, is directly related to the internal pressure, i.e. it is elastic, and can be readily controlled by regulating the pressure. The expansion of the distal section at failure within the second higher pressure range should not be more than about 25%, preferably not more than about 10% of the maximum diameter of the distal section at the maximum pressure within the first pressure range. Upon deflation, the distal section contracts to a diameter much smaller than the inflated diameters by means of elastic recoil and thereby provides for a rapid and complete deflation of the inflatable member.

In some of the presently preferred embodiments of the invention, only a distal portion of the outer tubular member is expandable. The length of the expandable distal section of this embodiment may range from about 5 mm to about 40 cm or more, and preferably ranges from about 2 to about 30 cm. In some instances the outer tubular member may be expandable along its entire length or any portion thereof.

As discussed above, the distal section of the outer tubular member is formed of polymer material which allows a significant elastic expansion when inflation fluid under elevated pressure is introduced therein into the annular lumen defined between the inner tubular member and the distal section of the outer tubular member. Beyond a particular expansion the distal section has a very limited compliance which ensures that the catheter shaft is not over inflated. The expandable portion of the outer tubular member is formed from a heat shrinkable thermoplastic material, particularly an irradiated cross-linked polymer material, which has been thermally treated to a temperature of not more than about 75° C, preferably not more than about 50° C, above or below the crystalline melting point of the polymer to provide the desired expansion. It is preferred to expand the expandable distal section at the thermal treatment temperature, cool and then heat shrink the expanded distal section at a temperature within about 45° C to about 80° C to a diameter much smaller than the expanded diameter. Particularly suitable materials for the expandable portion of the outer tubular member are polyolefinic ionomers selected from the group consisting of sodium, lithium or zinc ionomers such as Surlyn® by E.I. duPont, deNemours & Co. and particularly zinc ionomers 8020/IBE and 9020, sodium ionomers 8920 and 8940 and lithium ionomers 7930 and 7940. Other suitable heat shrinkable polymers include ethylene vinyl acetate such as ELVAX® sold by E.I. duPont, deNemours & Co.. The expandable distal section of the outer tubular member and the inflatable member may be formed from the same tubular element, with the different portions being given thermomechanical processing to provide the desired characteristics, as will be described subsequently. The ionomer material may be extruded at a temperature between about 121°C to about 260°C (about 250° F to about 500° F), preferably about 176°C to about 232°C (about 350° F to about 450° F).

The expandable distal section formed of the material as described above, keeps a relatively small outer diameter at low or ambient pressure, but, when subjected to inflation fluid at elevated pressures within the first pressure range, it elastically expands significantly thereby allowing the inflation fluid to readily flow to the interior of the inflatable member on the distal end of the catheter shaft and providing low inflation times. The expansion at pressures within the second pressure range is relatively limited because of the relatively noncompliant nature of the material. The little expansion which does occur can be easily controlled by the pressure levels of the inflation fluid. The elastic recoil of the expandable distal section upon reducing the pressure facilitates the rapid and complete deflation of the inflatable member and the distal section. The first pressure range may extend from about 709 to about 1215 kPa (about 7 to about 12 atmospheres) and the second pressure range may extend from about 101 to about 202 kPa (about 10 to about 20 atmospheres) with polyolefinic ionomer materials. Other materials may have other pressure ranges.

The invention provides substantial reductions in the transverse dimensions of the catheter shaft. Minimum transverse dimensions of expandable distal sections of the outer tubular members designed for coronary dilatation catheters are on the order of about 0.51 to about 1.5 mm (0.02-0.06 in). For peripheral arteries the transverse dimensions may be larger. Preferably, the deflated transverse dimension of the expandable distal section of the outer tubular member is essentially the same or just slightly smaller than the deflated transverse dimension of the inflatable member so as to present a distal portion of the catheter with a smooth exterior surface to facilitate the passage of the distal portion through tortuous anatomy. The catheter provides improved flexibility with little or no loss in pushability.

The improvements of the invention are applicable to a wide variety of intravascular catheters and to essentially all types of dilatation catheters with inflatable or expandable members, such as those described in the BACKGROUND OF THE INVENTION. These and other advantages of the invention will become more apparent from the following detailed description of the invention when taken in conjunction with accompanying exemplary drawings.

### BRIEF DESCRIPTION OF THE DRAWINGS

Fig. 1 is an elevational view, partially in section, of a balloon dilatation catheter embodying features of the invention.

Fig. 2 is a transverse cross-sectional view of the catheter shown in Fig. 1 taken along the lines 2-2.

Fig. 3 is a transverse cross-sectional view of the catheter shown in Fig. 1 taken along the lines 3-3.

Fig. 4 is a transverse cross-sectional view of the catheter shown in Fig. 1 taken along the lines 4-4.

Fig. 5 is a transverse cross-sectional view of the catheter shown in Fig. 1 taken along the lines 5-5.

Fig. 6 is an elevational view, partially in section, of a dilatation catheter embodying features of the invention which is adapted for rapid exchange during an intravascular procedure.

Fig. 7 is a transverse cross-sectional view of the catheter shown in Fig. 6 taken along the lines 7-7.

Fig. 8 is a transverse cross-sectional view of the catheter shown in Fig. 6 taken along the lines 8-8.

Fig. 9 is a transverse cross-sectional view of the catheter shown in Fig. 6 taken along the lines 9-9.

Fig. 10 is a transverse cross-sectional view of the catheter shown in Fig. 6 taken along the lines 10-10.

Fig. 11 is an elevational view, partially in section, of a dilatation catheter embodying features of the invention which is adapted to perfuse oxygenated blood distal to the inflated balloon of the catheter during an intravascular procedure.

Fig. 12 is a transverse cross-sectional view of the catheter shown in Fig. 11 taken along the lines 12-12.

Fig. 13 is a transverse cross-sectional view of the catheter shown in Fig. 11 taken along the lines 13-13.

Fig. 14 is a transverse cross-sectional view of the catheter shown in Fig. 11 taken along the lines 14-14.

Fig. 15 is a transverse cross-sectional view of the catheter shown in Fig. 11 taken along the lines 15-15.

Fig. 16 is a graphical representation of the expansion of two embodiments of the outer tubular member of the invention.

### DETAILED DESCRIPTION OF THE INVENTION

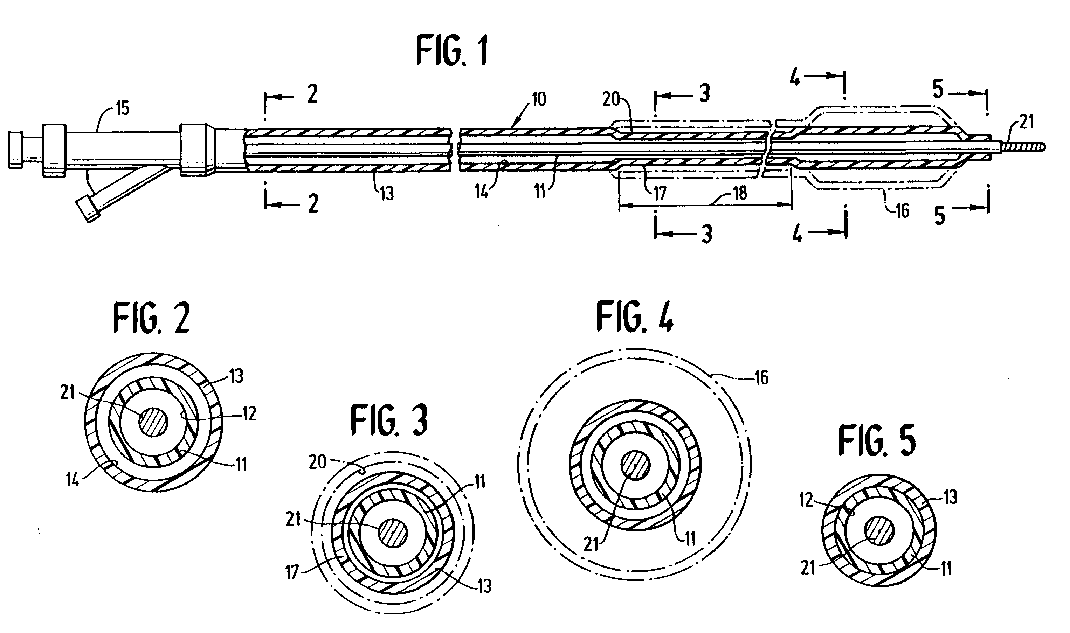

Figs. 1-5 schematically illustrate an over-the-wire dilatation catheter embodying features of the invention. The catheter includes an elongated catheter shaft 10 which has an inner tubular member 11 with an inner lumen 12 adapted to receive a guidewire, an outer tubular member 13 disposed about the inner tubular member and defining therebetween an annular inner lumen 14 which extends through the proximal portion of the catheter shaft. An adapter 15 is secured to the proximal ends of the inner and outer tubular members 11 and 13. An inflatable member or balloon 16 is formed as part of the outer tubular member 13 with the distal end of the inflatable member secured to the distal end of the inner tubular member 11. The inflatable member 16 and the distal expandable portion 17 of the shaft 10 may be formed from the same tubing as the proximal portion of the outer tubular member 13 as shown in Fig. 1 or they may be made separately and secured to the distal end of the proximal portion of the outer tubular member.

The inflatable member 16 preferably has essentially the same or, as shown in the drawings, slightly larger transverse dimensions as the distal expandable section 17 of the shaft 10. The distal section 17 along the length 18 when in the deflated condition forms a very thin annular inflation lumen 20 which is in fluid communication with the interior of the inflatable member 16 and the annular lumen 14. Upon inflation, the distal section 17 expands causing the transverse dimensions of the annular lumen 20 to increase. The inflated shape of the lumen 20 is shown in phantom in Figs. 1 and 3 and the inflated shape of the inflatable member 16 is shown in phantom in Figs. 1 and 4. Upon removal of inflation fluid both the distal section 17 and the inflatable member 16 elastically recoil back to essentially their original preinflation shapes with slightly larger radial dimension.

The use of the dilatation catheter shown in Figs. 1-5 generally follows conventional PTCA practices with over-the-wire dilatation catheters as described in the Background of the Invention. A guidewire 21 is backloaded into the inner lumen 12 of the inner tubular member 11 of the catheter body 10 and both the guidewire and the catheter are advanced together through a guiding catheter (not shown) which has been previously disposed within the patient's arterial system, with the distal end of the guiding catheter seated within the ostium of the desired coronary artery. The guidewire 21 is first advanced out the distal end of the guiding catheter into the patient's coronary anatomy until it extends beyond the lesion to be dilated, and then the dilatation catheter is advanced over the guidewire, which is being held in its position, until the inflatable member 16 on the dilatation catheter is properly disposed within the stenotic region so that the lesion can be dilated upon the inflation thereof. After the dilatation, the inflatable member 16 is deflated and the catheter and the guidewire are withdrawn from the patient. If further treatment or diagnosis is to be conducted, the guidewire 21 can be replaced with an exchange wire before removing the dilatation catheter so that the first catheter can be removed and another advanced into the desired location over the exchange wire or an extension wire can be attached to the proximal end of the guidewire in place to perform essentially the same function. See the discussion of exchange wires and extension wires in U.S. Patent 4,827,941 (Taylor *et al*.).

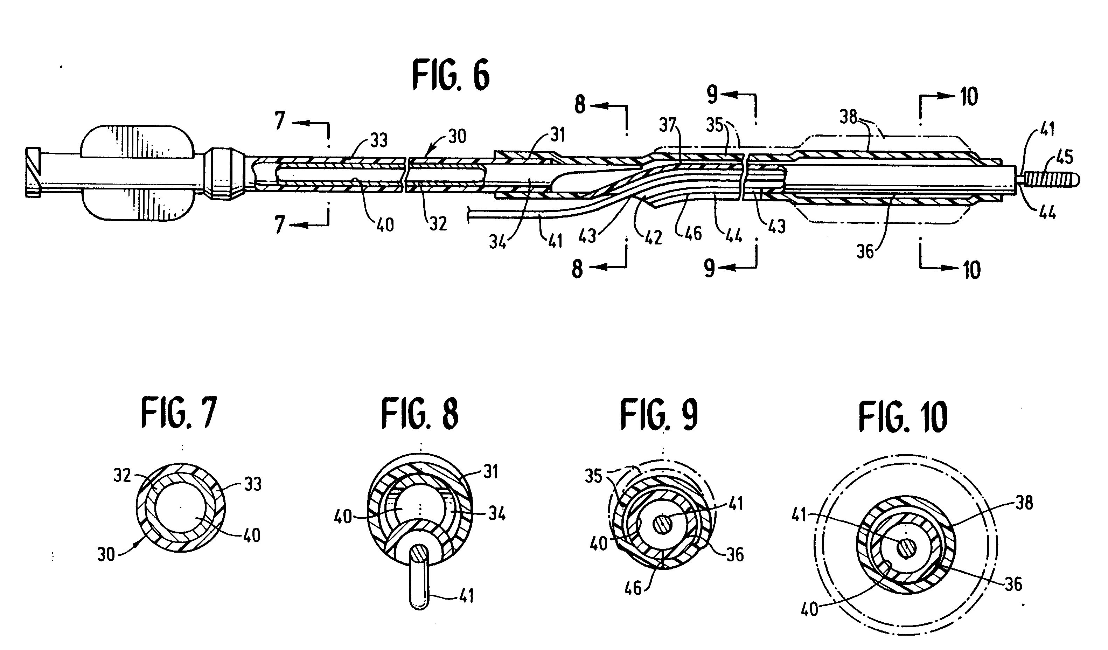

Figs. 6-10 schematically illustrate another dilatation catheter embodying features of the invention which is adapted for rapid exchange during an angioplasty procedure. The catheter includes a catheter shaft 30 having an outer tubular member 31 with a two layered proximal portion 32. The two layered proximal portion 32 has an outer plastic tubular jacket or coating 33 which fits tightly, *e.g.* is shrunk fit, onto a high strength tubular element 34 which may be formed of hypotubing. The outer tubular member 31 also includes an expandable distal section 35 which is disposed about the inner tubular member 36 with a very thin annular inflation lumen 37. As shown in phantom, upon the introduction of inflation liquid under pressure into the annular lumen 37, the distal section 35 expands along with the inflatable member or balloon 38 as shown in phantom in Figs 6, 9 and 10. Upon the withdrawal of the inflation fluid, the distal section 35 and the inflatable member 38 contract by elastic recoil to a much smaller outer dimension, usually just slightly larger than the original deflated dimensions. The distal section 35 may be the proximal skirt of the inflatable member 38, as shown in Fig. 6, or it may be a separate tubular member with the proximal skirt of the inflatable member 38 bonded to the distal end of the separate tubular member. The inflatable member 38 is secured by its distal end or skirt to the distal end of the inner tubular member 36 by suitable means such as heat shrinking or by heat or adhesive bonding. The annular inflation lumen 37 defined between the distal section 35 and the inner tubular member 36 is in fluid communication with inflation lumen 40 in the proximal section 32 and the interior of the inflatable member 38. The expanded inflated state of the distal section 35 shown in phantom provides for a very rapid inflation and deflation of the inflatable member 38.

Guidewire 41, which may be of conventional construction, extends through the inner lumen 42 of the inner tubular member 36 and out the proximal guidewire port 43 in the proximal end of the inner tubular member and a distal guidewire port 44 in the distal end of the inner tubular member. A flexible coil 45 is provided on the distal end of the guidewire 41.

The distal section 35 is bonded to the lower portion of the inner tubular member 36 by suitable means such as heat bonding or adhesives and a slit is 46 is provided through the bonded walls thereof from the proximal guidewire port 43 to a more distal location.

The inner tubular element 34 in the proximal portion of the shaft 30, onto which the outer plastic jacket 33 is secured, is preferably hypotubing and may be formed of conventional stainless steel or a NiTi alloy, particularly a NiTi alloy with superelastic properties.

The catheter construction of this embodiment with a relatively short inner lumen 42 adapted to slidably receive the guidewire 41, eliminates the need for using an exchange wire or a guidewire extension, as described in the Yock and Horzewski *et al.* patents. A peel-away slit 46 is preferably provided to facilitate the removal of the catheter from the proximal end of the guidewire 41 when the catheter is to be replaced or exchanged for another catheter. A dual lumen type catheter shaft construction such as described in Horzewski *et al*. may also be used in the portion of the catheter proximal to the proximal guidewire port 43.

There are at least two modes of inserting the dilatation catheter of the embodiment shown in Figs. 6-10 into the patient's coronary anatomy. The first method is for the most part the same as in the prior over-the-wire embodiment, namely, the guidewire 41 is preloaded into the short inner lumen 42 of the inner tubular member 36 and both are advanced through a guiding catheter (not shown) previously disposed within the patient's arterial system with the distal end of the guiding catheter seated within the ostium of a coronary artery as described in the Background of the Invention. The second mode, frequently called the "bare wire" technique, involves first advancing a guidewire 41 through the guiding catheter and out the distal end thereof until it is positioned within the patient's coronary artery across the lesion to be dilated. The proximal end of the guidewire 41, which is outside the patient, is backloaded, *i.e.* inserted, through the distal guidewire port 44 into the short inner lumen 42 of the inner tubular member 36 and advanced proximally therein until it exits the proximal guidewire port 43. The proximal end of the guidewire 40 is held in place while the catheter is advanced over the guidewire through the patient's vascular system until the dilatation balloon 38 on the catheter is positioned across the stenotic region so that the stenosis will be dilated upon the inflation of the balloon. After the dilatation of the lesion, the balloon 38 is deflated and then the catheter may be removed from the patient's artery. If other diagnostic or therapeutic procedures are contemplated or are possible, the catheter is slidably removed over the guidewire 41, leaving the guidewire in place, so that other catheters can be advanced over the in-place guidewire in a similar manner without the need for exchange wires or guidewire extensions.

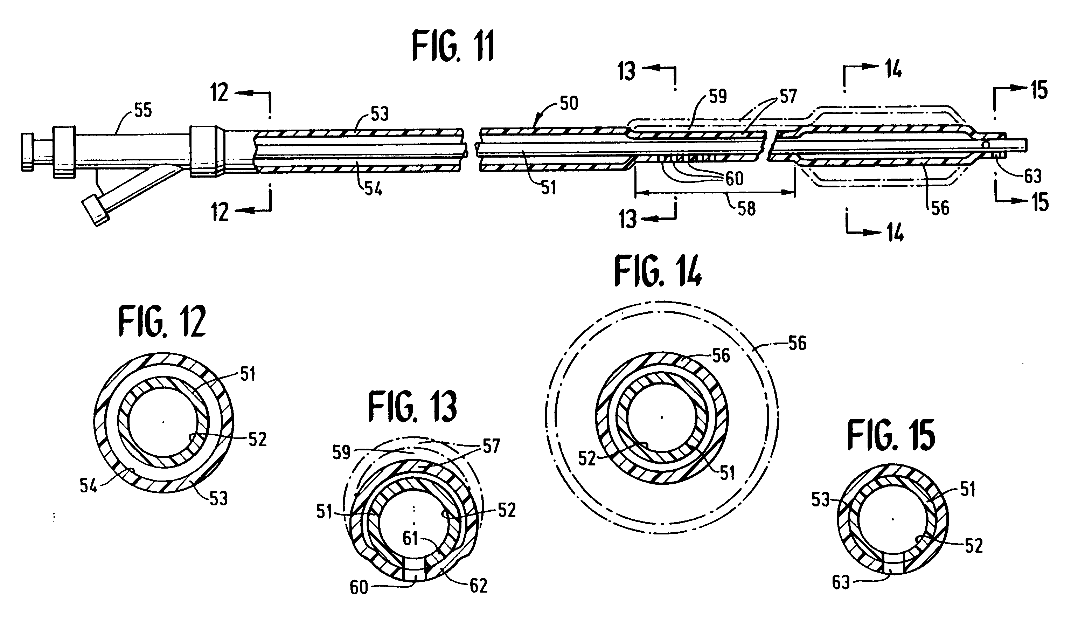

Figs. 11 through 15 illustrate yet another dilatation catheter embodying features of the invention which provides for the perfusion of blood distal to the catheter during the dilatation of a stenotic lesion. The catheter includes the catheter shaft 50, an inner tubular member 51 which has an inner lumen 52, an outer tubular member 53 which is disposed about the inner tubular member and which defines an annular inflation lumen 54 located between the inner and outer tubular members in the proximal portion of the catheter shaft. An adapter 55 is secured to the ends of the inner and outer members 51 and 53, and an inflatable member or balloon 56 is secured by its distal end to the distal end of the inner tubular member 51. The outer tubular member 53 has an expandable distal section 57 which is bonded along a length thereof to the exterior of the inner tubular member 51 as previously described in the embodiment shown in Figs 6-10, forming the crescent-shaped inflation lumen 58. The expandable distal section 57 expands upon the introduction of inflation liquid into the crescent shaped inflation lumen 58 and directs inflation fluid from the crescent-shaped lumen 58 to the interior of inflatable member 56.

The expandable distal section 57 of the catheter shaft 50, as shown in Figs. 11-15, has a plurality of perfusion ports 60 proximal to the inflatable member 56 which pass through the bonded walls of the inner and outer tubular members 51 and 53 respectively and which are in fluid communication with the inner lumen 52 of the inner tubular member 51. Additionally, one or more perfusion ports 61 are provided distal to the inflatable member 56 through the wall of the inner tubular member 51 and are in fluid communication with the inner lumen 52 extending therein. In this manner, when the inflatable member 56 is inflated during an angioplasty procedure, oxygenated blood is forced to pass through the proximal perfusion ports 60, through the inner lumen 52 and then out the distal perfusion ports 63 to provide oxygenated blood distal to the catheter and thereby avoid the generation of ischemic conditions in tissue downstream thereof or the aggravation of existing ischemic conditions. The transverse dimensions of the inner tubular member 51 within the bonded section are preferably larger than in the embodiments previously discussed to allow for an increased flow of blood therethrough.

The use of the embodiment shown in Figs. 11-15 is essentially the same as the embodiment shown in Figs. 1-5. The only essential difference is that the inflatable member 56 of the embodiment shown in Figs. 11-15 can be inflated for significantly longer periods, *e.g.* typically about 20-30 minutes but possibly up to 5 hours or more, than the first described embodiment because oxygenated blood is flowing to the tissue distal to the inflated balloon.

The dilatation catheter shown in Figs. 11-15 may be modified by providing a guidewire port at the proximal to the expandable distal section 57, as shown in Figs 6-10. However, the guidewire port should be spaced sufficiently far proximally from the portion of the bonded distal section 57 having the perfusion ports 60 so that the guidewire can be pulled proximally and remain within the inner lumen 52 while the balloon 56 is inflated during the dilatation but not interfere with the flow of blood through the perfusion ports 60 and 63 and the inner lumen 52. Alternatively, means can be provided within the inner lumen 52 proximal to the perfusion ports 60 to grasp the guidewire to hold the guidewire in position while blood passes through the inner lumen 52. After the angioplasty procedure is completed, the guidewire can then be advanced distally through the inner lumen 52 and out the distal end thereof in order to maintain access to the lesion in case further treatment or diagnosis is necessary or desirable.

The use of the catheter with both perfusion ports and a proximal guidewire port as described above is essentially the same as the use of the dilatation catheter illustrated in Figs. 6-10, but with the additional advantage that long term dilatations are possible.

The above described catheters may be made by conventional techniques well known to those skilled in the art. The various components of the catheters and guidewires of the invention, unless otherwise discussed, can be formed from a wide variety of conventional materials. The catheter shaft proximal to the expandable distal section may be made from polyethylene, polyimide, polyvinyl chloride, zinc, lithium and sodium ionomers and other suitable plastic materials. The hypotubing may be formed of stainless steel, NiTi superelastic alloys or other suitable materials.

The dimensions of the catheters of the invention generally follow the dimensions of conventional intravascular catheters. For coronary angioplasty the length is typically about 135 cm and the outer diameter of the outer tubular member is about 0.51 mm to about 1.5 mm (about 0.02 to about 0.06 inch). In a presently preferred embodiment, the distal expandable section is long enough (*e.g.* preferably about 10 to about 40 cm) to ensure that it is the only portion of the catheter shaft proximal to the balloon which exits the guiding catheter and enters the patient's coronary anatomy during intravascular procedures. The transverse dimensions of the catheter may be larger with catheters for use in peripheral arteries and other locations. The thicknesses of the expandable distal section depends upon the desired mechanical properties for this material.

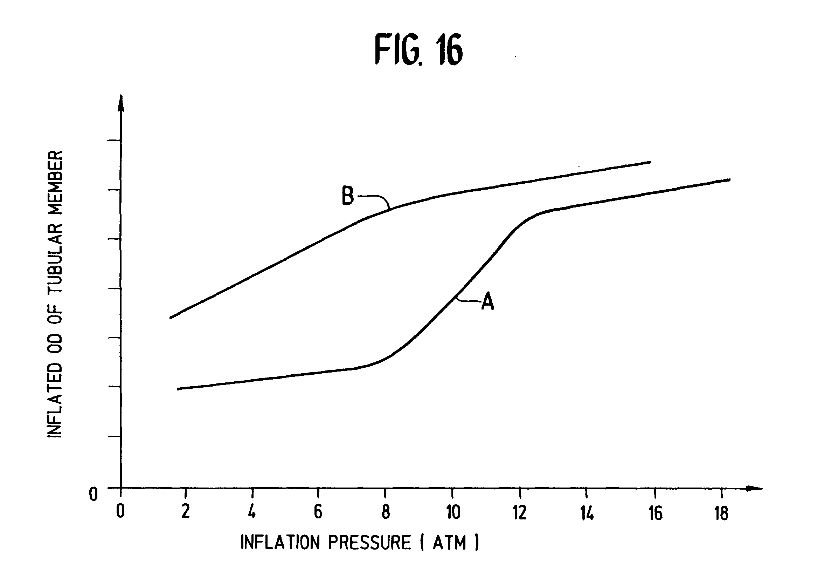

The following example is given to further illustrate features of the invention. An outer tubular member for a dilatation catheter was prepared having a structure essentially as shown in FIGS. 1-3 and made of Surlyn® (8020 grade), a zinc ionomer supplied by the E.I. duPont, deNemours & Company. The outer tubular member had an OD of about 0.94 mm (0.037 inch) and an ID of about 0.61 mm (0.025 inch), *i.e.* a wall thickness of about 0.15 mm (0.006 inch) over essentially its entire length. The outer tubular member 14 was irradiated (gamma radiation) at a level of about 45 to 55 mrads in order to cross-link essentially the entire polymeric tube. The distal portion of the polymerized tubular member which was to become the expandable distal portion was subjected to a thermal treatment at about 121°C (250° F) for a period of about 20 seconds while applying tension in the longitudinal direction in order to develop a significant level of longitudinal orientation in the inflatable portion. After the thermally treated inflatable portion of the tubular member had cooled to room temperature, the dilatation catheter 10 was assembled. The relationship between the outer diameter of the expandable section and the fluid pressure is represented in FIG. 16 by curve A. As is evident, at pressures from about one to about 12 atmospheres, the change in the outer diameter of the inflatable section is negligible, indicating that the material has little compliance within this pressure range. At pressures from about 12 to about 14 atmospheres there is a transition region wherein the material exhibits substantial expansion in the elastic mode. At internal pressures above about 14 atmospheres the expansion of the expandable section 17 was quite small, i.e. it was relatively noncompliant, and the expansion was essentially linear with respect to the interior pressure within the inflatable section.

The following example is given to illustrate another presently preferred embodiment of the invention. Pellets of a lithium olefinic ionomer such as Surlyn 7930 or 7940 are extruded at a temperature of about 176°C (350° F) to about 230°C (450° F) into tubular stock. Upon exiting from the extrusion die, the tubular stock is quenched in a trough of cool water, preferably about 7-13°C (45-55° F), in order to obtain a highly amorphous material. The cooled tubular product is stabilized at about 4 to 26°C (40° F to 80° F,) typically about 15°C (60° F) for about 2 to about 6 hours, typically about 4 hours. The portion of the stabilized tubular product which is to be formed into the expandable distal section is then irradiated at about 45 to about 75 Mrads, preferably about 55 to about 65 Mrads. The tubular product is then subjected to an internal pressure of about 3.45x10⁵ - 5.86x10⁵ Pa (50 to about 85 psi), preferably about 4.14x10⁵ to about 5.17x10⁵ Pa (60 to about 75 psi.) at a temperature of about 107°C (225° F) to about 121°C (250° F) to expand or blow the irradiated portion of the tubular member. The expanded tubular section is assembled with other components into a catheter and then it is heat treated at about,55° C to about 65° C for about 10 to about 30 minutes to heat shrink the expanded section to a diameter the same or slightly larger than its original diameter. This embodiment provides an expandable distal section which expands significantly in the elastic mode within pressure range much lower than the first discussed embodiment.

The curve B in Fig. 16 illustrates the expansion of the distal section formed in the above manner at various interior pressures. The starting point of curve B, as shown, is after expanded section has been inflated with sufficient inflation liquid to be relatively wrinkle free. In the first stage, starting at about 405 kPa (4 atmospheres) and extending to about 410 kPa (8 atmospheres), the expansion rate of the inflatable member is relatively high and in the elastic mode. At a pressure level of about 410 kPa (8 atmospheres) and beyond, the expansion of the inflatable member is relatively low, i.e. the inflatable member is relatively noncompliant. Upon deflation of the inflated member, the diameter of the inflatable member follows the expansion rates shown in the drawing to essentially the starting point.

Various modifications can be made to the present invention. For example, with the aforementioned preferred embodiment only the distal portion of the outer tubular member 14 that was to form the expandable distal section 17 was subjected to the heat treatment. If desired, the entire outer tubular member 14 can be given a thermal treatment to develop the characteristics of elastic expansion but the exterior of the non-inflatable portion of the outer tubular member may be provided with an inelastic jacket or coating so that only the inflatable section 16 inflates when subjected to internal pressure. Other modifications include forming the inflatable section of an outer tubular member in accordance with the invention and secure the inflatable section to a catheter shaft of different material or the same material with differing properties. The preexpansion of the expandable distal section during the thermal treatment before heat shrinking the expanded section also decreases the rate of increase of the expansion, but it does not substantially change the second pressure range in which the material is relatively noncompliant.

While the invention has been described herein primarily in terms of catheters for coronary angioplasty, the invention may be employed in a wide variety of catheters for dilatation of various body lumens, e.g. peripheral arteries and male urethras for treatment of prostatic hyperplasia.

## Claims

1. An intraluminal catheter comprising:
an elongated catheter shaft (10) having proximal and distal (17) portions, the shaft having an inner tubular member (11) and an outer tubular member (13) which is disposed about said inner tubular member (11) to define therebetween an annular inner lumen (14) which extends through the proximal portion of the catheter shaft wherein the distal portion of the outer tubular member has a first configuration in which it forms an inflation lumen (20), and a second configuration in which it is collapsed about the inner tubular member; and
an inflatable member (16) at a distal end of the distal portion of the catheter shaft, wherein said inflation lumen (20) is in fluid communication with the interior of the inflatable member and the annular lumen (14) **characterized in that**:-
the distal portion of the outer tubular member (13) expands substantially elastically from said second configuration upon inflation to said first configuration in a first pressure range, and expands very little in said first configuration in a second pressure range higher than the first pressure range.

2. The intraluminal catheter of claim 1 wherein the outer tubular member (13) has the inner tubular member (11) disposed within the distal expandable portion thereof which together with the distal expandable portion defines the expandable inflation lumen (20).

3. The intraluminal catheter of claim 2 wherein the expandable inflation lumen (20) has an annular transverse cross-section.

4. The intraluminal catheter of claim 2 wherein the inner tubular member (11) and the expandable distal portion (17) of the outer tubular member are bonded along a length thereof and the expandable inflation lumen defined therebetween has a crescent-shaped transverse cross-section.

5. The intraluminal catheter of claim 2 wherein the inner tubular member has a guidewire receiving inner lumen (12) extending therein to a guidewire port in the distal end thereof.

6. The intraluminal catheter of claim 1 including means on the proximal end of the shaft to direct inflation fluid at elevated pressure from a source through an inner lumen (12) in the catheter shaft to expand the expandable distal section (20) to facilitate the flow of inflation fluid to the interior of the balloon.

7. The intraluminal catheter of claim 1 wherein the outer diameter of the expandable distal section of the outer tubular member upon inflation expands from 5 to 50% of the deflated outer diameter.

8. The intraluminal catheter of claim 1 wherein the outer diameter of the expandable distal section (17) of the outer tubular member upon inflation expands from about 10 to about 30% of the deflated outer diameter.

9. The intraluminal catheter of claim 1 wherein the inflatable member (16) and the expandable distal section (17) of the outer tubular member are formed in a one piece construction from the same polymeric material.

10. The intraluminal catheter of claim 1 wherein the inflatable member (16) is formed independently of the outer tubular member and has a proximal end secured to a distal portion of the expandable distal section (17) of the outer tubular member.

11. The intraluminal catheter of claim 1 wherein the inflatable member (16) has a distal end secured to the distal end of the inner tubular member (11) which extends through the interior of the inflatable member.

12. The intraluminal catheter of claim 1 including a guidewire port in the catheter shaft in fluid communication with the inner lumen (12) of the inner tubular member which is spaced proximately from the inflatable member and a substantial distance from the proximal end of the catheter shaft.

13. The intraluminal catheter of claim 4 wherein at least one perfusion portion is provided through the bonded portion of the inner tubular member (11) and the expandable distal section which is in fluid communication with the inner lumen within the inner tubular member.

14. A method of forming an outer tubular member (13) for an intraluminal catheter in accordance with any preceding claim, the outer tubular member (13) having an expandable distal portion and an inflatable member (16) at a distal end of the distal portion, the method comprising:
a) extruding through a die an elongated tubular member (13) formed of a polymer material selected from the group consisting of sodium, lithium and zinc ionomers and blends thereof at elevated temperatures;
b) quenching the extruded tubular member as it exits from the die;
c) irradiating first and second portions of the tubular member which are to be formed into the expandable distal portion and the inflatable member (16) respectively, to cross-link the polymer material thereof;
d) heat treating the first and second portions of the tubular member at a temperature 75°C above or 75°C below the crystalline melting point of the polymer material; and
e) expanding by inflation the first and second portions and then cooling and heat shrinking the first and second portions, the second portion being expanded to a larger diameter than the first portion.

15. The method of claim 14, the first and second portions are expanded at a temperature 50°C above or 50°C below the crystalline melting point of the polymer material.

16. The method of claim 14 wherein the polymer material is extruded at a temperature between 121°C (250°F) and 260°C (500°F).

17. The method of claim 14 wherein the extruded tubular member (13) is quenched in an aqueous bath at a temperature between 4°C (40°F) and 15°C (60°F).

18. The method of claim 17 wherein the quenched tubular member (13) is stabilized at a temperature between 50°C and 70°C.

19. The method of claim 17 wherein the portions of the tubular member which are to be formed into the inflatable member (16) and the expandable distal portion (17) are irradiated with 50 to 70 Mrad of electron beam radiation.

20. The method of claim 19 wherein the irradiated tubular member is heat treated at a temperature of 110°C (230°F) to 121°C (250°F).

21. The method of claim 20 wherein the first portion of the heat treated tubular member which is to be formed into the expandable distal portion (17) is expanded by inflation fluid at a pressure between 3.45 x 10⁵-5.86x⁵pa (50 and 85 psi) while the tubular member is maintained at the heat treatment temperature.

22. the method of claim 21 wherein the expanded tubular member is heat shrunk by heating to a temperature of 45°C to 80°C for 5 to 60 minutes.

## Revendications

1. Cathéter intraluminal comprenant :
un axe allongé (10) de cathéter comportant des parties proximale et distale (17), l'axe comportant un élément tubulaire intérieur (11) et un élément tubulaire extérieur (13) qui est disposé autour dudit élément tubulaire intérieur (11) pour définir, entre eux, une lumière intérieure annulaire (14) qui s'étend à travers la partie proximale de l'axe de cathéter, dans lequel la partie distale de l'élément tubulaire extérieur a une première configuration dans laquelle elle forme une lumière (20) de gonflage, et une seconde configuration dans laquelle elle est écrasée autour de l'élément tubulaire intérieur ; et
un élément gonflable (16) situé à une extrémité distale de l'axe de cathéter, dans lequel ladite lumière (20) de gonflage est en communication fluidique avec l'intérieur de l'élément gonflable et la lumière annulaire (14), **caractérisé en ce que** :
la partie distale de l'élément tubulaire extérieur (13) se dilate sensiblement élastiquement lors du gonflage, dans une première plage de pression, de ladite seconde configuration à ladite première configuration et, dans une seconde plage de pression plus élevée que la première plage de pression, ne se dilate que très peu dans ladite première configuration.

2. Cathéter intraluminal selon la revendication 1, dans lequel l'élément tubulaire extérieur (13) comporte l'élément tubulaire intérieur (11) disposé à l'intérieur de sa partie distale pouvant se dilater qui, conjointement avec la partie distale pouvant se dilater, définit la lumière (20) de gonflage pouvant se dilater.

3. Cathéter intraluminal selon la revendication 2, dans lequel la lumière (20) de gonflage pouvant se dilater a une section transversale annulaire.

4. Cathéter intraluminal selon la revendication 2, dans lequel l'élément tubulaire intérieur (11) et la partie distale pouvant se dilater (17) de l'élément tubulaire extérieur sont liés le long de sa longueur et la lumière de gonflage pouvant se dilater définie entre eux a une section transversale en forme de croissant.

5. Cathéter intraluminal selon la revendication 2, dans lequel l'élément tubulaire intérieur comporte une lumière intérieure (12) de réception de fil de guidage s'étendant dans celui-ci vers un orifice de fil de guidage situé dans son extrémité distale.

6. Cathéter intraluminal selon la revendication 1, incluant un moyen, situé sur l'extrémité proximale de l'axe, servant à diriger un fluide de gonflage sous pression élevée d'une source à travers une lumière intérieure (12) dans l'axe de cathéter, destiné à dilater la section distale pouvant se dilater (20) dans le but de faciliter l'écoulement d'un fluide de gonflage vers l'intérieur du ballonnet.

7. Cathéter intraluminal selon la revendication 1, dans lequel, lors du gonflage, le diamètre extérieur de la section distale pouvant se dilater de l'élément tubulaire extérieur se dilate de 5 à 50 % par rapport au diamètre extérieur dégonflé.

8. Cathéter intraluminal selon la revendication 1, dans lequel, lors du gonflage, le diamètre extérieur de la section distale pouvant se dilater (17) de l'élément tubulaire extérieur se dilate d'environ 10 à environ 30 % par rapport au diamètre extérieur dégonflé.

9. Cathéter intraluminal selon la revendication 1, dans lequel l'élément gonflable (16) et la section distale pouvant se dilater (17) de l'élément tubulaire extérieur sont formés en une structure d'une seule pièce dans la même matière polymère.

10. Cathéter intraluminal selon la revendication 1, dans lequel l'élément gonflable (16) est formé indépendamment de l'élément tubulaire extérieur et comporte une extrémité proximale fixée à une partie distale de la section distale pouvant se dilater (17) de l'élément tubulaire extérieur.

11. Cathéter intraluminal selon la revendication 1, dans lequel l'élément gonflable (16) comporte une extrémité distale fixée à l'extrémité distale de l'élément tubulaire intérieur (11) qui s'étend à l'intérieur de l'élément gonflable.

12. Cathéter intraluminal selon la revendication 1, incluant un orifice de fil de guidage dans l'axe de cathéter en communication fluidique avec la lumière intérieure (12) de l'élément tubulaire intérieur qui est espacé, de manière proximale, de l'élément gonflable et à distance substantielle de l'extrémité proximale de l'axe de cathéter.

13. Cathéter intraluminal selon la revendication 4, dans lequel au moins une partie de perfusion est prévue à travers la partie liée de l'élément tubulaire intérieur (11) et de la section distale pouvant se dilater, laquelle est en communication fluidique avec la lumière intérieure à l'intérieur de l'élément tubulaire intérieur.

14. Procédé de formation d'un élément tubulaire extérieur (13) pour un cathéter intraluminal selon l'une quelconque des revendications précédentes, dans lequel l'élément tubulaire extérieur (13) comporte une partie distale pouvant se dilater et un élément gonflable (16) au niveau d'une extrémité distale de la partie distale, le procédé comprenant :
a) l'extrusion, à travers une filière, d'un élément tubulaire allongé (13) formé de matière polymère choisie à partir du groupe constitué d'ionomères de sodium, de lithium et de zinc, et de mélanges de ceux-ci à des températures élevées ;
b) le trempage de l'élément tubulaire extrudé à sa sortie de la filière ;
c) l'application d'un rayonnement aux première et seconde parties de l'élément tubulaire à former, respectivement, en la partie d'extrémité pouvant se dilater et l'élément gonflable (16), pour liaison croisée de leur matière polymère ;
d) le traitement à la chaleur des première et seconde parties de l'élément tubulaire à une température de 75°C au-delà ou de 75°C en deçà du point de cristallisation de la matière polymère ; et
e) la dilatation par gonflage des première et seconde parties et ensuite refroidissement et thermorétrécissement des première et seconde parties, la seconde partie étant dilatée à un diamètre plus grand que la première partie.

15. Procédé selon la revendication 14, dans lequel les première et seconde parties sont dilatées à une température de 50°C au-delà ou de 50°C en deçà du point de cristallisation de la matière polymère.

16. Procédé selon la revendication 14, dans lequel on extrude la matière polymère à une température située entre 121°C (250 °F) et 260°C (500 °F).

17. Procédé selon la revendication 14, dans lequel l'élément tubulaire extrudé (13) est trempé dans un bain aqueux à une température qui se situe entre 4°C (40 °F) et 15°C (60 °F).

18. Procédé selon la revendication 17, dans lequel l'élément tubulaire trempé (13) est stabilisé à une température qui se situe entre 50°C et 70°C.

19. Procédé selon la revendication 17, dans lequel les parties de l'élément tubulaire à former en l'élément gonflable (16) et la partie distale pouvant se dilater (17) font l'objet d'une projection de rayonnement de faisceau d'électrons de 50 à 70 Mrad.

20. Procédé selon la revendication 19, dans lequel l'élément tubulaire ayant fait l'objet d'un rayonnement est traité à la chaleur à une température qui se situe entre 110°C (230 °F) et 121°C (250 °F).

21. Procédé selon la revendication 20, dans lequel la première partie de l'élément tubulaire traité à la chaleur à former en la partie distale pouvant se dilater (17) est dilatée par fluide de gonflage à une pression qui se situe entre 3,45 x 10⁵ et 5,86 x 10⁵ Pa (50 et 85 psi), tandis que l'élément tubulaire est maintenu à la température de traitement à la chaleur.

22. Procédé selon la revendication 21, dans lequel l'élément tubulaire dilaté est thermorétréci par chauffage à une température qui se situe entre 45°C et 80°C pendant 5 à 60 minutes.

## Patentansprüche

1. Intraluminaler Katheter mit:
einem länglichen Katheterschaft (10), der proximale und distale (17) Abschnitte hat, wobei der Schaft ein inneres rohrförmiges Element (11) und ein äußeres rohrförmiges Element (13) hat, das um das innere rohrförmige Element (11) angeordnet ist, um dazwischen ein ringförmiges Innenlumen (14) zu begrenzen, das sich durch den proximalen Abschnitt des Katheterschafts erstreckt, wobei der distale Abschnitt des äußeren rohrförmigen Elements eine erste Konfiguration hat, in der er ein Aufblählumen (20) bildet, und eine zweite Konfiguraton, in der er um das innere rohrförmige Element zusammengesunken ist; und
einem aufblähbaren Element (16) an einem distalen Ende des distalen Abschnitts des Katheterschafts, wobei das Aufblählumen (20) in fluidmäßiger Verbindung ist mit dem Inneren des aufblähbaren Elements und dem ringförmigen Lumen (14), **dadurch gekennzeichnet, daß**:-
der disiale Abschnitt des äußeren rohrförmigen Elements (13) sich im wesentlichen elastisch beim Aufblähen von der zweiten Konfiguration in die erste Konfiguration in einem ersten Druckbereich dehnt und sich sehr geringfügig in der ersten Konfiguration dehnt in einem zweiten Druckbereich, der höher ist als der erste Druckbereich.

2. Intraluminaler Katheter nach Anspruch 1, bei welchem das äußere rohrförmige Element (13) das innere rohrförmige Element (11) innerhalb seines distalen, dehnbaren Abschnitts angeordnet hat, was zusammen mit dem distalen, dehnbaren Abschnitt das dehnbare Aufblählumen (20) definiert.

3. Intraluminaler Katheter nach Anspruch 2, bei welchem das dehnbare Aufblählumen (20) einen ringförmigen, transversalen Querschnitt hat.

4. Intraluminaler Katheter nach Anspruch 2, bei welchem das innere ringförmige Element (11) und der dehnbare, distale Abschnitt (17) des äußeren rohrförmigen Elements entlang einer seiner Längen verbunden sind und das dazwischen definierte, dehnbare Aufblählumen einen mondförmigen, transversalen Querschnitt hat.

5. Intraluminaler Katheter nach Anspruch 2, bei welchem das innere rohrförmige Element ein Innenlumen (12) zur Aufahme eines Führungsdrahtes hat, der sich darin zu einer Führungsdrahtöffnung in dessen distalem Ende erstreckt.

6. Intraluminaler Katheter nach Anspruch 1 mit Mitteln an dem proximalen Ende des Schafts, über die Aufblähfluid bei erhöhtem Druck von einer Quelle durch ein Innenlumen (12) in dem Katherterschaft gefühxt wird, um den dehnbaren distalen Abschnitt (20) zu dehnen zwecks Vereinfachung des Stroms an Aufblähfluid zum Inneren des Ballons.

7. Intraluminaler Katheter nach Anspruch 1, bei welchem sich der Außendurchmesser des dehnbaren distalen Abschnitts des äußeren rohrförmigen Elements beim Aufblähen auf 5 bis 50% des zusammengssunkenen Außendurchmessers dehnt.

8. Intraluminaler Katheter nach Anspruch 1, bei welchem sich der Außendurchmesser des dehnbaren distalen Abschnitts (17) des äußeren rohrförmigen Elements beim Aufblähen auf etwa 10 bis etwa 30% des zusammengesunkenen Außendurchmessers dehnt.

9. Intraluminaler Katheter nach Anspruch 1, bei welchem das aufblähbare Element (16) und der dehnbare distale Abschnitt (17) des äußeren rohrförmigen Elements in einstückigem Aufbau aus dem gleichen Polymerwerkstoff gebildet sind.

10. Intraluminaler Katheter nach Anspruch 1, bei welchem das aufblähbare Element (16) unabhängig von dem äußeren rohrförmigen Element gebildet ist und ein proximales Ende hat, das an einem distalen Abschnitt des dehnbaren, distalen Abschnitts (17) des äußeren rohrförmigen Elements befestigt ist.

11. Intraluminaler Katheter nach Anspruch 1, bei welchem das aufblähbare Element (16) ein distales Ende hat, das an dem distalen Ende des inneren rohrförmigen Elements (11) befestigt ist, das sich durch das Innere des aufblähbaren Elements erstreckt.

12. Intraluminaler Katheter nach Anspruch 1 mit einer Führungsdrahtöffnung in dem Katheterschaft, die in fluidmäßiger Verbindung mit dem Innenlumen (12) des inneren rohrförmigen Elements steht, das eng beabstandet ist von dem aufblähbaren Element und in wesentlichem Abstand steht von dem proximalen Ende des Katheterschafts.

13. Intraluminaler Katheter nach Anspruch 4, bei welchem wenigstens ein Perfusionsabschnitt durch den Verbindungsabschnitt des inneren rohrförmigen Elements (11) und dem dehnbaren distalen Abschnitt vorgesehen ist, der in fluidmäßiger Verbindung mit dem Innenlumen innerhalb des inneren rohrförmigen Elements ist.

14. Verfahren zur Ausbildung eines äußeren rohrförmigen Elements (13) für einen intraluminalen Katheter in Übereinstimmung mit einem der vorstehenden Ansprüche, wobei das äußere rohrförmige Element (13) einen dehnbaren, distalen Abschnitt und ein aufblähbares Element (16) an einem distalen Endes des distalen Abschnitts hat, wobei das Verfahren aufweist:
a) durch eine Form extrudieren eines länglichen rohrförmigen Elements (13), das aus einem Polymerwerkstoff gebildet ist, der ausgewählt ist aus der Gruppe bestehend aus Natrium-, Lithium- und Zinkionomeren und deren Gemischen bei erhöhten Temperaturen;
b) abschrecken des extrudierten rohrförmigen Elements bei seinem Austritt aus der Form;
c) bestrahlen erster und zweiter Abschnitte des rohrförmigen Elements, die zu dem dehnbaren, distalen Abschnitt bzw. dem aufblähbaren Element (16) ausgebildet werden sollen, um deren Polymerwerkstoff zu vernetzen;
d) wärmebehandeln der ersten und zweiten Abschnitte des rohrförmigen Elements bei einer Temperatur von 75°C über oder 75°C unter dem kristallinen Schmelzpunkt des Polymerwerkstoffs; und
e) durch Aufblasen, dehnen der ersten und zweiten Abschnitte und dann kohlen und wärmeschrumpfen der ersten und zweiten Abschnitte, wobei der zweite Abschnitt auf einen größeren Durchmesser gedehnt wird als der erste Abschnitt.

15. Verfahren nach Anspruch 14, wobei die ersten und zweiten Abschnitte gedehnt werden bei einer Temperatux von 50°C oberhalb oder 50°C unterhalb des kristallinen Schmelzpunktes des Polymerwerkstoffs.

16. Verfahren nach Anspruch 14, bei welchem der Polymerwerkstoff extrudiert wird bei einer Temperatur von zwischen 121°C (250°F) und 260°C (500°F).

17. Verfahren nach Anspruch 14, bei welchem das extrudierte rohrförmige Element (13) abgeschreckt wird in einem wäßrigen Bad bei einer Temperatur von zwischen 4°C (40°F) und 15°C (60°F).

18. Verfahren nach Anspruch 17, bei welchem das abgeschreckte rohrförmige Element (13) stabilisiert wird bei einer Temperatur von zwischen 50°C und 70°C.

19. Verfahren nach Anspruch 17, bei welchem die Abschnitte des rohrförmigen Elements, die zu dem aufblähbaren Element (16) und dem dehnbaren distalen Abschnitt (17) ausgebildet werden sollen, bestrahlt werden mit 50 bis 70 Mrad einer Elektronenstrahlstrahlung.

20. Verfahren nach Anspruch 19, bei welchem das bestrahlte rohrförmige Element wärmebehandelt wird bei einer Temperatux von 110°C (230°F) bis 121°C (250°F).

21. Verfahren nach Anspruch 20, bei welchem der erste Abschnitt das wärmebehandelten rohrförmigen Elements, der zu dem dehnbaren distalen Abschnitt (17) ausgebildet werden soll, gedehnt wird durch ein Autblähfluid bei einem Druck von zwischen 3,45 x 10⁵-5,86 x 10⁵pa (50 und 85 psi), während das rohrförmige Element auf der Wärmebehandlungs-temperatur gehalten ist.

22. Verfahren nach Anspruch 21, bei welchem das gedehnte rohrförmige Element wärmegeschrumpft ist durch Erwärmen auf eine Temperatur von 45°C bis 80°C über 5 bis 50 Minuten.
